# EUROPEAN PATENT APPLICATION

(11) **EP 2 436 411 A1**
(43) Date of publication of application: **04.04.2012**
(21) Application number: 11007381.4
(22) Date of filing: 09.09.2011
(51) Int. Cl.: A61M 5/28

(54) **Packaging of delivery device for aseptic filling and distribution**

(30) Priority: 30.09.2010 US 894482
(71) Applicant: Tyco Healthcare Group, LP, Mansfield, MA 02048 (US)
(72) Inventor: Melvin, Finke, DeLand, FL 32720 (US); Foster, John, K., Port Orange, FL 32128 (US); Morefield, Ellen, Deland, FL 32720 (US); Parker, Jonathan, G., Carmel, IN 46032 (US)
(74) Representative: Olsson, Carl H.S.

(57) **Abstract**

A syringe assembly (10) includes a syringe body (12) including a distal end (13). The syringe body (12) defines a fluid chamber (18) therein. The fluid chamber (18) includes a lower end (17). A septum (19) is disposed at the distal end (13) of the syringe body (12) and is configured to sealingly close the lower end (17) of the fluid chamber (18). The syringe body (12) and the septum (19) are formed as a single, molded piece.

## Description

### 1. Technical Field

The present disclosure relates to syringes and, more particularly, to medical syringe assemblies such as disposable syringes and prefilled syringes.

### 2. Discussion of Related Art

A conventional syringe typically includes a hollow barrel configured to slideably receive a plunger having a piston supported on a distal end thereof. The plunger is received in the hollow barrel and partially extends through a proximal end thereof. In use, as the plunger is translated relative to the hollow syringe barrel, the piston is also translated to thereby aspirate and/or dispense fluid into/out of the hollow barrel. The syringe barrel may be provided with a mounting collar for threadedly engaging the hub of a needle cannula. The hub and the needle cannula may be connected to one another or may be maintained separately from the syringe barrel until shortly prior to use.

Syringes have been developed for use as disposable syringes that are discarded after a single administration and which include a selectively attachable/detachable plunger assembly. A single-use syringe prevents reuse of the syringe to minimize exposure of patients to HIV, hepatitis and other blood-borne pathogens.

Liquid pharmaceuticals and other injectable materials are often stored in rigid containers or vials which can be accessed using a needle cannula. Typically, great care must be taken when a needle cannula of a syringe is used in conjunction with a vial containing a pharmaceutical to be aspirated into the syringe.

In the case of pre-filled syringes, the syringe is provided with a hollow barrel containing a fluid and closed at the proximal end with a plunger slideably disposed within the bore of the barrel and closed at the distal end with a septum secured to the barrel by a crimped-on aluminum collar. Typically, in pre-filled syringes, the needle does not contact the fluids contained in the barrel when the syringe is in storage or shipment. To use the syringe, the proximal end of the needle cannula must penetrate the sealed septum such that fluid communication is established between the fluid chamber of the barrel and the proximal end of the needle.

### SUMMARY

The present disclosure relates to a syringe assembly including a syringe body including a distal end. The syringe body defines a fluid chamber therein. The fluid chamber includes a lower end. A septum is disposed at the distal end of the syringe body and is configured to sealingly close the lower end of the fluid chamber. The syringe body and the septum are formed as a single, molded piece.

The present disclosure also relates to an injection device including an injection needle, a needle/hub assembly configured to support the injection needle, and a syringe body including a distal end. The needle/hub assembly is coupled to the distal end of the syringe body. The injection device also includes a septum disposed at the distal end of the syringe body. The syringe body and the septum are formed as a single, molded piece.

The present disclosure also relates to an injection device including an injection needle defining a fluid channel, a stopper member including a body defining a lumen therein, and a syringe body defining a fluid chamber therein and including a distal end defining an opening. The body of the stopper member includes a distal end and a proximal end, wherein the proximal end includes at least one aperture defined therein and the distal end is configured to receive a proximal end of the injection needle such that the fluid channel of the injection needle communicates with the lumen of the stopper member. The stopper member is partially disposed within the opening and configured to be movably positionable from a first configuration wherein the lumen is not disposed in fluid communication with the fluid chamber to a second configuration wherein the lumen is disposed in fluid communication with the fluid chamber.

### BRIEF DESCRIPTION OF THE DRAWINGS

Objects and features of the presently-disclosed syringe assemblies will become apparent to those of ordinary skill in the art when descriptions of various embodiments thereof are read with reference to the accompanying drawings, of which:

FIG. 1 1 is a perspective view of a syringe assembly according to an embodiment of the present disclosure;

FIG. 2A is an enlarged, perspective view of a portion of the syringe assembly of FIG. 1 shown with the hub assembly in a first configuration according to an embodiment of the present disclosure;

FIG. 2B is an enlarged, perspective view of a portion of the syringe assembly of FIG. 1 shown with the hub assembly in a second configuration according to an embodiment of the present disclosure;

FIG. 3 is a perspective view of another embodiment of a syringe assembly in accordance with the present disclosure;

FIG.4A is an enlarged, perspective view of a portion of yet another embodiment of a syringe assembly shown in a first configuration in accordance with the present disclosure;

FIG. 4B is an enlarged, perspective view of the portion of the syringe assembly of FIG. 4A shown in a second configuration according to an embodiment of the present disclosure;

FIG. 5 is an enlarged, perspective view of an alternative embodiment of the portion of the syringe assembly shown in FIG. 4A in accordance with the present disclosure;

FIG. 6A is a perspective view of a portion of still another embodiment of a syringe assembly shown in a first configuration in accordance with the present disclosure; and

FIG. 6B is a perspective view of the portion of the syringe assembly of FIG. 6A shown in a second configuration according to an embodiment of the present disclosure.

### DETAILED DESCRIPTION

Hereinafter, embodiments of a syringe assembly are described with reference to the accompanying drawings. Like reference numerals may refer to similar or identical elements throughout the description of the figures. As shown in the drawings and as used in this description, and as is traditional when referring to relative positioning on an object, the term "proximal" refers to that portion of the apparatus, or component thereof, closer to the user and the term "distal" refers to that portion of the apparatus, or component thereof, farther from the user.

This description may use the phrases "in an embodiment," "in embodiments," "in some embodiments," or "in other embodiments," which may each refer to one or more of the same or different embodiments in accordance with the present disclosure. For the purposes of this description, a phrase in the form "A/B" means A or B. For the purposes of the description, a phrase in the form "A and/or B" means "(A), (B), or (A and B)".

As it is used in this description, "pharmaceuticals" generally refers to any chemical substance intended for use in the medical diagnosis, cure, treatment, and/or prevention of disease. As it is used in this description, "plastic" generally refers to any of a wide variety of resins or polymers with different characteristics and uses.

Embodiments of the presently-disclosed syringe assembly are suitable for use as disposable syringes and prefilled syringes. Embodiments of the presently-disclosed syringe assembly include a syringe barrel (also referred to herein as a syringe body) including a distally-positioned septum, wherein the syringe barrel and the septum are formed as a single, molded piece, which advantageously eliminates the need for a crimped-on aluminum collar often used in conventional hypodermic syringes.

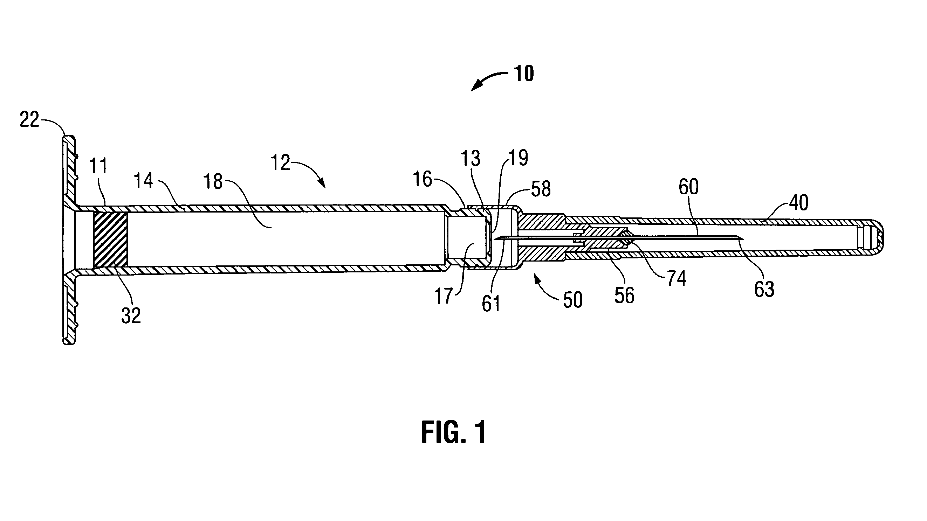

FIGS. 1 through 2B illustrate one embodiment of the presently-disclosed syringe assembly shown generally as 10. Syringe assembly 10 is suitable for use in pre-filled applications, and may be a single-use assembly which is disposable after use on a subject. Syringe assembly 10 includes a syringe body 12, a piston or plunger 32 positioned at the proximal end 11 of the syringe body 12, a hub/needle assembly 50 positioned at the distal end 13 of the syringe body 12, and a needle protector or sheath 40. Syringe assembly 10, or portions thereof, may be transparent or semi-transparent, which may allow a user to visually inspect material disposed within the syringe body 12.

Syringe body 12 defines a fluid chamber or reservoir 18 therein. Fluid reservoir 18 generally includes a lower end 17. Syringe body 12 includes a distally-positioned septum 19, which may be formed integrally with the syringe body 12. In embodiments, the syringe body 12 and the septum 19 are formed as a single, molded piece by a suitable molding process, such as injection molding.

Fluid reservoir 18 is generally configured to contain a material, such as, liquid pharmaceuticals or other injectable materials, to be dispensed and/or aspirated. Septum 19 is configured to sealingly close the lower end 17 of the fluid reservoir 18. In embodiments, the fluid reservoir 18 may have an internal volume of about 0.3 ml (milliliters) to about 100 ml, although other fluid reservoir volumes are envisioned. In some embodiments, fluid reservoir 18 is filled with a solution. In certain applications, syringe assembly 10 may contain a flushing solution, e.g., saline or the like. Alternatively, syringe assembly 10 may contain medicaments, including antibiotics, pain medication, therapeutic drugs, heparin or the like. Plunger 32 is configured to be slideably received within fluid reservoir 18 of the syringe body 12. In one embodiment, the plunger 32 is formed from an elastomeric material.

In some embodiments, the syringe body 12 is made of plastic, such as, transparent polypropylene. Syringe body 12 may be constructed of nearly any polymeric material. Adjacent a proximal end of the syringe body 12 is a peripheral flange 22, which extends perpendicularly beyond the periphery of the syringe body 12. Syringe flange 22 may be integrally formed with the syringe body 12.

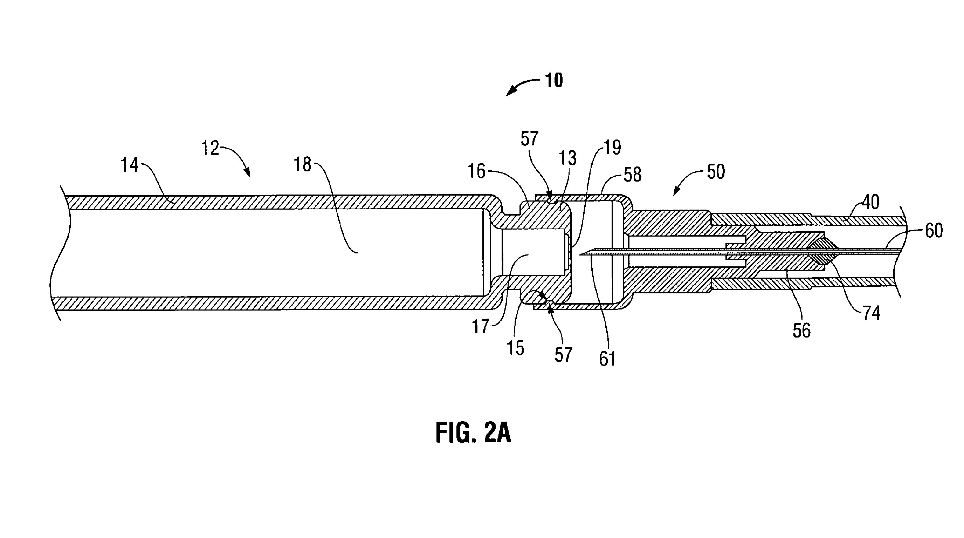

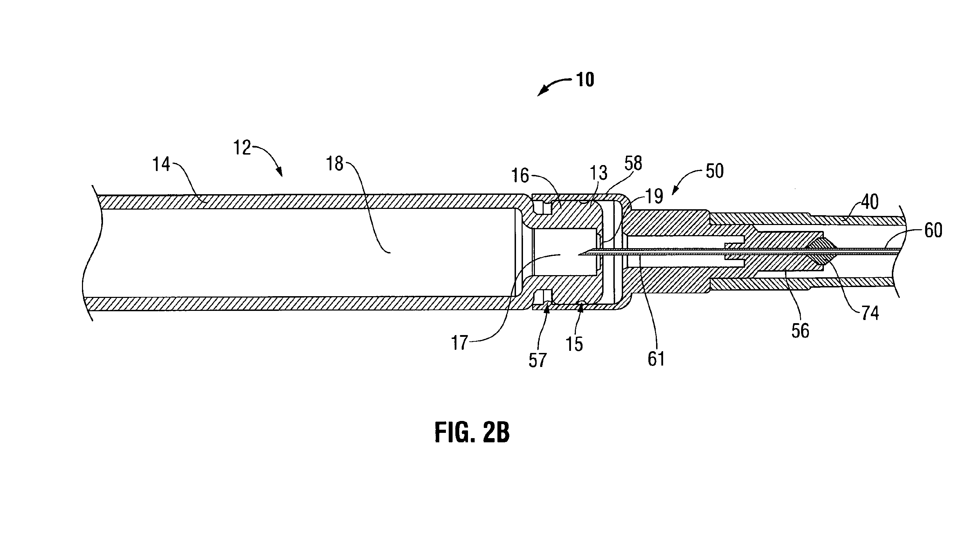

In the embodiment illustrated in FIGS. 1, 2A and 2B, the syringe body 12 includes a generally cylindrical body portion 14 and a head portion 16 defined at a distal end of the body portion 14. In some embodiments, the distally-positioned septum 19 is formed integrally with the head portion 16. In some embodiments, head portion 16 and the septum 19 are formed as a single, molded piece by a suitable molding process, such as injection molding.

In some embodiments, the body portion 14 is formed of a first material and the head portion 16 is formed of a second material. In some embodiments, the head portion 16, or portions thereof, may be formed of a softer material than the material used to form the body portion 14. The second material may be selected to be a material pierceable by a needle cannula.

Hub/needle assembly 50 includes a skirt 58, a needle support 56, and a needle cannula 60 having a proximal end 61 and a distal end 63. Needle cannula 60 may be a stainless steel needle secured within the needle support 56 in a known manner (e.g., by a suitable adhesive 74) at a distal end of the hub/needle assembly 50. Skirt 58 is generally configured to have a circumference greater than the circumference of the head portion 16 of the syringe body 12. Skirt 58 includes an engagement member 57 configured to snap-fit, or otherwise engage, a correspondingly dimensioned channel 15 defined in the head portion 16.

Needle protector or sheath 40 is configured to receive the needle 60 therein and includes an open end configured to be releaseably coupleable with the distal end of the hub/needle assembly 50. Sheath 40 may be formed of flexible plastic materials, rigid plastic materials, or other material or materials. Sheath 40 may have a hollow, substantially cylindrical shape. Walls of the sheath 40 may be spaced apart from the needle 60 such that the sheath 40 does not contact needle 60.

As cooperatively shown in FIGS. 2A and 2B, the hub/needle assembly 50 is configured and movably positionable, e.g., relative to head portion 16 of the syringe body 12, such that the proximal end 61 of the needle 60 pierces the septum 19 by movement of the hub/needle assembly 50 in a proximal direction from a first configuration wherein the proximal end 61 of the needle 60 is disposed distal to the septum 19, to a second configuration wherein the proximal end 61 of the needle cannula 60 is disposed proximal to the septum 19 and in fluid communication with the lower end 17 of the fluid chamber 18.

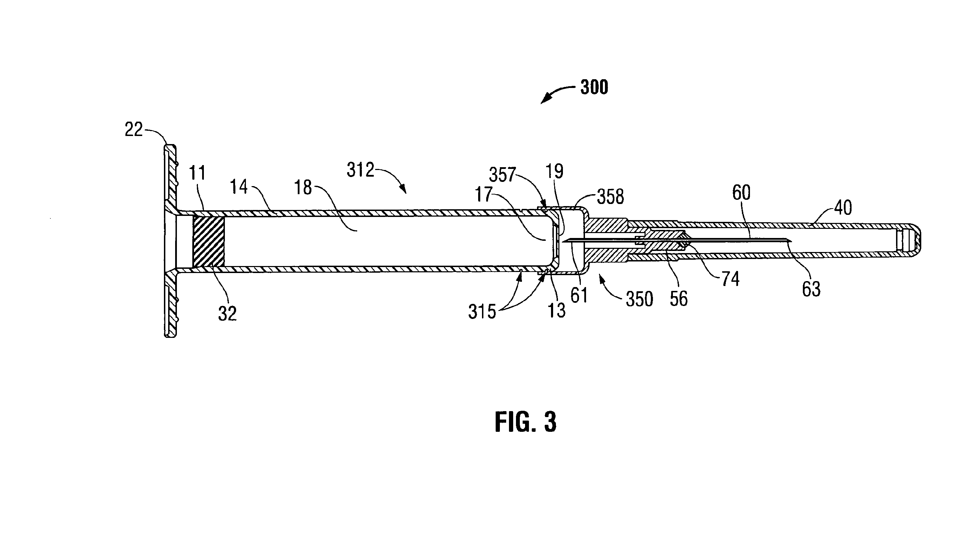

FIG. 3 illustrates another embodiment of the presently-disclosed syringe assembly shown generally as 300. Syringe assembly 300 is similar to the syringe assembly 10 of FIGS. 1 through 2B except for the configuration of the syringe barrel 312 and the shape and/or size of the skirt 358 of the hub/needle assembly 350. More particularly, the head portion 16 of the syringe assembly 10 is omitted in the simpler, syringe barrel 312, which may reduce manufacturing costs, particularly, tooling and material costs, for the syringe assembly 300 as compared to the syringe assembly 10.

Syringe assembly 300 includes a syringe body 312, a piston or plunger 32 positioned at the proximal end 11 of the syringe body 312, a hub/needle assembly 350 positioned at a distal end of the syringe body 312, and the needle protector or sheath 40. Skirt 358 is generally configured to have a circumference greater than the circumference of the syringe body 312. Skirt 358 includes an engagement member 357 configured to snap-fit, or otherwise engage, a correspondingly dimensioned channel 315 defined in the syringe body 312. Syringe body 312 may include one or more channels 315 defined therein. In the embodiment illustrated in FIG. 3, two channels 315 are shown at a spaced apart distance from one another to allow engagement of the engagement member 357 therewith when the hub/needle assembly 350 is in a first configuration (e.g., wherein the proximal end 61 of the needle 60 is disposed distal to the septum 19) and a second configuration (e.g., wherein the proximal end 61 of the needle 60 is disposed proximal to the septum 19).

Syringe body 312 includes a septum 19 disposed at the distal end 13 of a generally cylindrical body 14 of the syringe body 312. Septum 19 is configured to sealingly close the lower end 17 of the fluid chamber 18 defined by the body 14. In some embodiments, syringe body 312 and the septum 19 are integrally formed as a unitary structure. In some embodiments, syringe body 312 and the septum 19 are formed as a single, molded piece by a suitable molding process, such as injection molding.

As illustrated in FIG. 3, the hub/needle assembly 350 is configured and positioned, for example relative to the syringe body 312, such that the proximal end of the needle 60 pierces the septum 19 by movement of the hub/needle assembly 350 in a proximal direction.

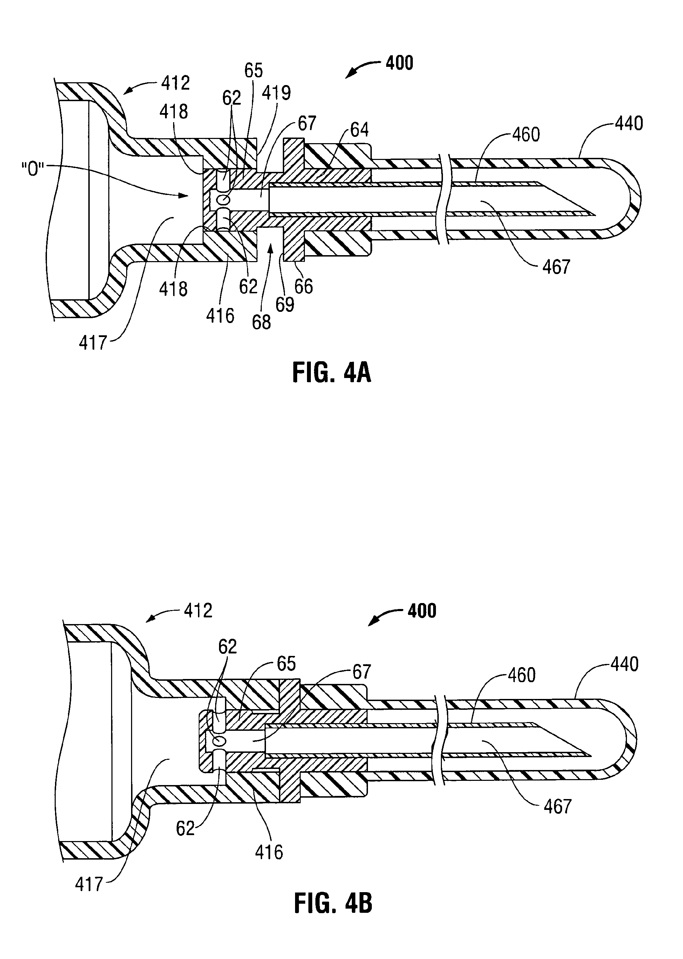

FIGS. 4A and 4B illustrate another embodiment of the presently-disclosed syringe assembly shown generally as 400. Syringe assembly 400 includes a syringe body 412, a stopper member 68, a needle cannula 460, and a needle protector or sheath 440. Syringe body 412 includes a neck portion 416 defining a fluid chamber 417 therein and including inner walls 418 defining a passage or opening "O" at the distal end of the fluid chamber 417.

Stopper member 68 includes a body 64 defining a lumen 67 therein. The distal end of the body 64 is configured to receive the proximal end of the needle cannula 460 such that the lumen 67 is disposed in fluid communication with the needle channel 467. Body 64 may have a generally cylindrical shape, although other shapes are envisioned. Needle cannula 460 may be fastened to the body 64 using any suitable fastening techniques, e.g., physical, chemical or mechanical, including adhesives. At its proximal end, the body 64 includes a head portion 65 including one or more apertures 62 defined therein, wherein each aperture 62 is disposed in fluid communication with the lumen 67. Head portion 65 is configured to sealingly engage the inner walls 418 of the neck portion 416 of the syringe body 412.

In some embodiments, the body 64 includes a flange 66, which may have a generally annular shape. As cooperatively shown in FIGS. 4A and 4B the flange 66 is disposed at a pre-determined spaced apart distance from the head portion 65 such that movement of the body 64 in a proximal direction positions the proximal side 69 of the flange 66 adjacent to the distal end 419 of the neck portion 416 while positioning the apertures 62 in fluid communication with the fluid chamber 417. The relative positions, size and/or shape of the apertures 62, the head portion 65, and the flange 66 may be varied from the embodiment depicted in FIGS. 4A and 4B.

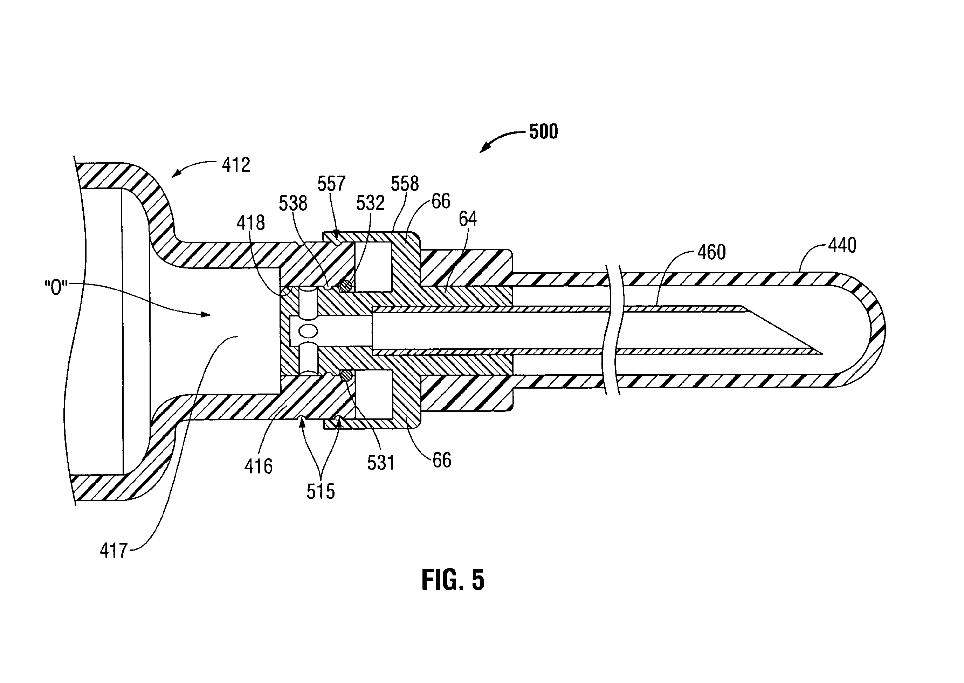

FIG. 5 illustrates another embodiment of the presently-disclosed syringe assembly shown generally as 500. Syringe assembly 500 is similar to the syringe assembly 400 shown in FIG. 4A, except that syringe assembly 500 includes a skirt 558 configured with an engagement member 557 for engaging channels 515 defined in the neck portion 416 of the syringe body 412, and an O-ring 532 and a protrusion 538 associated with the inner wall 418 of the neck portion 416. Skirt 558, engagement member 557, and channels 515 are similar to the skirt 358, engagement member 357, and channels 315 shown in FIG. 3, respectively, and further description thereof is omitted in the interests of brevity.

In the embodiment illustrated in FIG. 5, an O-ring 532 is provided about the opening "O" defined by the inner wall 418 of the neck portion 416. O-ring 532 is configured to provide a fluid seal between the inner wall 418 and the body 64. Inner wall 418 may include a recess 531 defined therein for receiving a portion of the O-ring 532. Recess 531, or portions thereof, may be provided with an adhesive layer (not shown) for fixedly securing the O-ring 532. O-ring 532 may be formed of any suitable material, e.g., an elastomeric material. One or more protrusions 538 may additionally, or alternatively, be disposed on the inner wall 418 and configured to apply compressive force to body 64 (e.g., head portion 65) to inhibit flow of a material, such as liquid pharmaceuticals, from the fluid chamber 417, particularly when under pressure from a plunger/piston (not shown), around the outer periphery of the body 64.

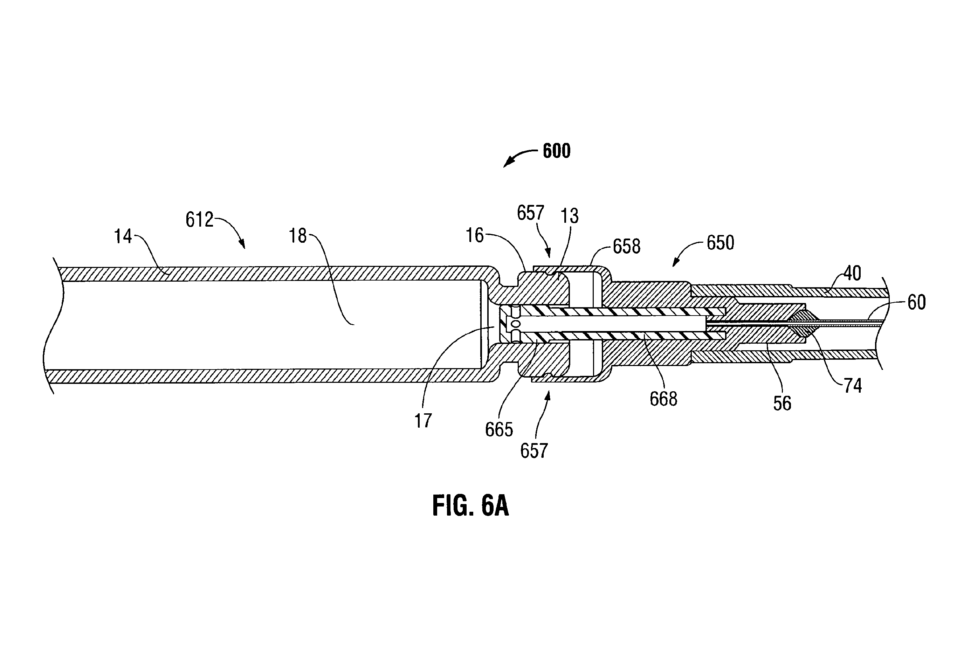

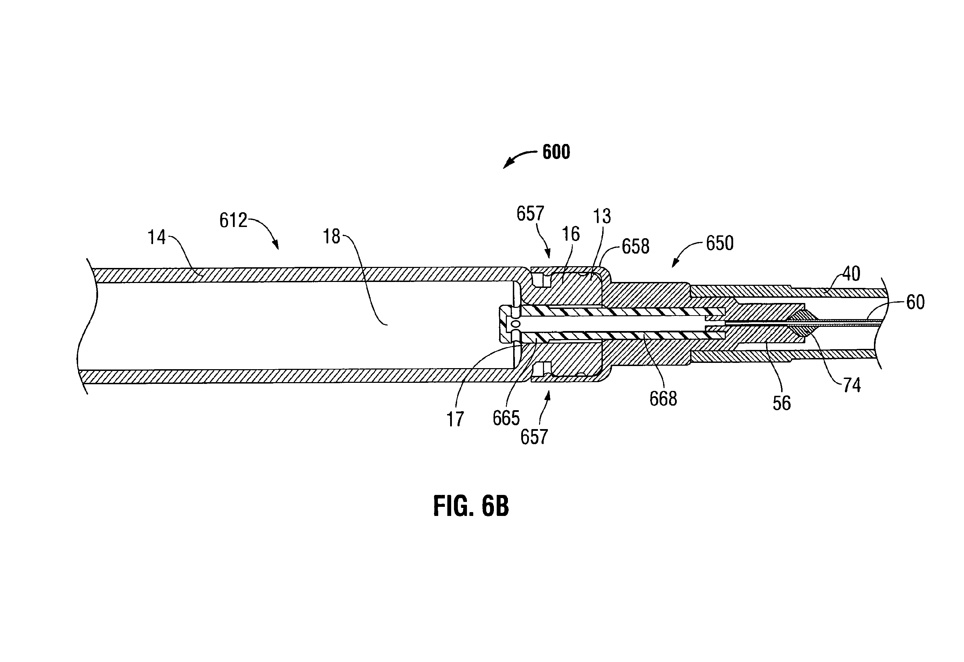

FIGS. 6A and 6B illustrate another embodiment of the presently-disclosed syringe assembly shown generally as 600. Syringe assembly 600 includes a syringe body 612 and a hub/needle assembly 650 that includes a stopper member 668 and a needle cannula 60. Syringe body 612 is similar to the syringe body 12 shown in FIGS. 1 through 2B, except that septum 19 is not included in syringe body 612. Further description thereof is omitted in the interests of brevity. Stopper member 668 is similar to the stopper member 68 shown in FIGS. 4A and 4B, except that flange 66 is not included in stopper member 668. Further description thereof is omitted in the interests of brevity. Hub/needle assembly 650 includes a skirt 658 including an engagement 657 configured to snap-fit, or otherwise engage the head portion 16 of the syringe body 612.

As cooperatively shown in FIGS. 6A and 6B, the hub/needle assembly 650 is configured and movably positionable, e.g., relative to head portion 16 of the syringe body 612, such that the head portion 665 of the stopper member 668 enters the fluid chamber 18 by movement of the hub/needle assembly 650 in a proximal direction from a first configuration wherein the needle cannula 60 is not in fluid communication with the fluid chamber 18, to a second configuration wherein the needle cannula 60 is in fluid communication with the fluid chamber 18.

The above-described syringe assemblies may be suitable for use as disposable syringes and/or prefilled syringes.

The above-described syringe assemblies which include a syringe body having a distally-positioned septum integrally formed with the syringe body or a repositionable stopper member eliminate the need for a crimped-on aluminum collar often used in conventional hypodermic syringes, e.g., to secure a rubber septum to a glass syringe barrel.

Although embodiments have been described in detail with reference to the accompanying drawings for the purpose of illustration and description, it is to be understood that the inventive processes and apparatus are not to be construed as limited thereby. It will be apparent to those of ordinary skill in the art that various modifications to the foregoing embodiments may be made without departing from the scope of the disclosure.

## Claims

1. A syringe assembly, comprising:
a syringe body including a distal end;
a fluid chamber defined within the syringe body, the fluid chamber including a lower end; and
a septum disposed at the distal end of the syringe body and configured to sealingly close the lower end of the fluid chamber, wherein the syringe body and the septum are formed as a single, molded piece.

2. The syringe assembly of claim 1, wherein the syringe body has a generally cylindrical shape.

3. The syringe assembly of claim 1, wherein the syringe body includes a first portion formed of a first material and a second portion formed of a second material.

4. The syringe assembly of claim 3, wherein the first portion is a body portion having a generally cylindrical shape.

5. The syringe assembly of claim 4, wherein the second portion is a head portion defining a distal end of the body portion.

6. The syringe assembly of claim 5, wherein the second material is a softer material than the first material.

7. The syringe assembly of claim 6, wherein the second material is selected to be a material pierceable by a needle cannula.

8. The syringe assembly of claim 7, further comprising:
a needle/hub assembly coupled to the head portion, the needle/hub assembly including:
a skirt;
a needle support; and
a cannula needle coupled to the needle support.

9. The syringe assembly of claim 8, wherein the skirt includes an engagement member configured to engage the head portion.

10. The syringe assembly of claim 8, wherein the needle/hub assembly is configured such that the cannula needle pierces the septum by movement of the hub/needle assembly in a proximal direction.

11. An injection device, comprising:
an injection needle;
a needle/hub assembly configured to support the injection needle;
a syringe body including a distal end, wherein the needle/hub assembly is coupled to the distal end of the syringe body; and
a septum disposed at the distal end of the syringe body, wherein the syringe body and the septum are formed as a single, molded piece.

12. The injection device of claim 11, wherein the needle/hub assembly is configured such that the injection needle pierces the septum by movement of the hub/needle assembly in a proximal direction.

13. The injection device of claim 11, wherein the syringe body defines a fluid chamber therein, the fluid chamber including a lower end.

14. The injection device of claim 13, wherein the septum is configured to sealingly close the lower end of the fluid chamber.

15. An injection device, comprising:
an injection needle defining a fluid channel;
a stopper member including a body defining a lumen therein, the body including a distal end and a proximal end, wherein the proximal end includes at least one aperture defined therein and the distal end is configured to receive a proximal end of the injection needle such that the fluid channel of the injection needle communicates with the lumen of the stopper member; and
a syringe body defining a fluid chamber therein and including a distal end defining an opening, wherein the stopper member is partially disposed within the opening and configured to be movably positionable from a first configuration wherein the lumen is not disposed in fluid communication with the fluid chamber to a second configuration wherein the lumen is disposed in fluid communication with the fluid chamber.

16. The injection device of claim 15, wherein the body includes a flange disposed a pre-determined spaced apart distance from the proximal end of the body such that movement of the body in a proximal direction positions a proximal side of the flange adjacent to a distal end of the syringe body while positioning the at least one aperture in fluid communication with the fluid chamber.
